# FASCICULE DE BREVET EUROPEEN

(11) **EP 0 686 386 B1**
(45) Date de publication et mention de la délivrance du brevet: **30.08.2000**
(21) Numéro de dépôt: 95401325.6
(22) Date de dépôt: 08.06.1995
(51) Int. Cl.: A61K 7/00, A61K 7/48

(54) **Composition cosmétique ou dermatologique sous forme d'une dispersion, aqueuse et stable, de particules de gel cubique à base de phytantriol et contenant un agent tensioactif à chaine grasse en tant qu'agent dispersant et stabilisant**
Dermatologische und kosmetische Zusammensetzung aus eine stabile wässrige Dispersion von Phytantriol enthaltende Gelpartikels mit einem langkettiges Tensid als Dispergens und Stabilisator
Cosmetic and dermatologic composition comprising a stable aqueous dispersion of phytantriol-based gel particles containing a long-chain surfactant as dispersant and stabilizer

(30) Priorité: 08.06.1994 FR 9407031
(43) Date de publication de la demande: 13.12.1995
(73) Titulaire: L'OREAL, S.A., 75008 Paris (FR)
(72) Inventeur: Ribier, Alain, F-75001 Paris (FR); Biatry, Bruno, F-75012 Paris (FR)
(74) Mandataire: Stalla-Bourdillon, Bernard

(56) Documents cités:
- WO-A-84/02076
- WO-A-93/06921
- DATABASE WPI Week 9216 Derwent Publications Ltd., London, GB; AN 92-127237 & JP,A,04 069 316 (KURARAY) , 4 Mars 1992

## Description

La présente invention a pour objet une composition cosmétique ou dermatologique à usage topique se présentant sous forme d'une dispersion, aqueuse et stable, de particules de gel cubique à base de 3,7,11,15-tétraméthyl 1,2,3-hexadecanetriol ou phytantriol et contenant un agent tensioactif hydrosoluble à chaîne grasse en tant qu'agent dispersant et stabilisant.

Les divers principes actifs généralement présents dans les compositions cosmétiques et dermatologiques peuvent être de nature hydrophile ou lipophile et requièrent donc des véhicules compatibles avec leur nature respective. Il est d'usage courant, lorsque l'on souhaite formuler simultanément des principes actifs hydrophiles et lipophiles, de réaliser des compositions dites biphases, c'est-à-dire comprenant une phase aqueuse et une phase lipidique. Les exemples les plus classiques de ces types de composition sont bien entendu les émulsions quelles soient de type eau-dans-l'huile ou huile-dans-l'eau. Toutefois, il est bien connu que lors de son application, l'émulsion "casse", libérant ainsi brutalement les principes actifs qu'elle contenait. Ceux-ci sont alors absorbés par la peau, leur vitesse de pénétration variant considérablement selon leur nature. Ainsi, les émulsions paraissent être un véhicule peu satisfaisant lorsque l'on souhaite la présence simultanée de principes actifs de nature différente dans au moins une couche déterminée de la peau, en particulier en vue de l'obtention d'un effet de synergie entre les différents principes actifs.

Dans US 5.151.272, il a été décrit des compositions et en particulier des gels à libération contrôlée, notamment transdermique, de principes actifs. De telles compositions se présentent sous forme de phase cristal liquide, notamment cubique, constituée d'un mélange d'eau, de monooléine et éventuellement associée à de la phosphatidylcholine.

Les phases cubiques se présentent généralement sous forme de gels transparents, visqueux et isotropes en lumière polarisée. Elles sont organisées d'une manière bipolaire en domaines hydrophiles et lipophiles distincts, en contact étroit et formant un réseau tridimensionnel thermodynamiquement stable. Une telle organisation a été décrite notamment dans "La Recherche, vol. 23, pp. 306-315, Mars 1992" et dans "Lipid Technology, vol. 2, n° 2, pp. 42-45, Avril 1990". Selon l'arrangement des domaines hydrophile et lipophile, la phase cubique est dite de type normal ou inverse. Le terme de gel cubique utilisé dans la présente invention regroupe bien entendu les différents types de phase cubique.

Il a également été décrit dans JP 92-69316, l'obtention de gels aqueux transparents à partir de phytantriol et d'eau.

Cependant, ces compositions sous forme de gel ayant une structure phase cristal liquide cubique présentent une forte viscosité ainsi qu'un toucher collant, rèche et poisseux et donc des caractéristiques sensorielles peu satisfaisantes.

Il a été décrit dans WO 93/06921, des dispersions de particules constituées d'une phase interne non lamellaire sous forme de phase cristal liquide cubique ou hexagonale ou de phase cristal liquide L3 et d'une phase externe, lamellaire, cristal liquide ou L3. Une telle structure est obtenue par association de monooléine et d'eau ou de monoléine, de phosphatidylcholine et d'eau pour former une phase cristal liquide homogène qui est ensuite fragmentée, en présence d'un solvant et généralement d'un agent tensioactif tel que notamment des polymères blocs amphiphiles comme les Poloxamers encore appelés Pluronic, pour former une dispersion de particules.

Toutefois, ces dispersions présentent un inconvénient majeur à savoir leur manque de stabilité. En effet, on a constaté l'apparition de cristaux lorsque la dispersion était conservée à basse température (+4°C). Ce phénomène matérialise la perte de la structure, cubique et continue, des particules, et donc la perte des propriétés de séquestration/libération attendues de ces dispersions.

On a maintenant constaté de façon surprenante et inattendue qu'il était possible d'obtenir des compositions cosmétiques ou dermatologiques stables contenant, sous forme dispersée des particules de gel cubique, présentant à la fois des domaines hydrophile et lipophile permettant d'y inclure des principes actifs hydrophiles et lipophiles, celles-ci ayant un réseau de mailles réduites et contrôlables permettant de moduler la disponibilité des principes actifs séquestrés. Ces compositions constituent donc un support idéal pour des principes actifs de polarités opposées et incompatibles ou d'activités complémentaires voire synergiques. Ces compositions présentent non seulement une excellente stabilité mais également un toucher sensoriel très satisfaisant, et sont obtenues en dispersant dans un milieu aqueux des particules de gel cubique à base de phytantriol en présence d'au moins un agent tensioactif hydrosoluble à chaîne grasse.

La présente invention a donc pour objet une composition cosmétique ou dermatologique, à usage topique, sous forme d'une dispersion, aqueuse et stable, de particules de gel cubique à base de phytantriol comprenant :
(a) de 0,1 à 15 % en poids de 3,7,11,15-tétraméthyl 1,2,3-hexadecanetriol ou phytantriol par rapport au poids total de la composition, et
(b) de 0,1 à 3 % en poids d'un agent dispersant et stabilisant par rapport au poids total de la composition, ledit agent étant choisi parmi les agents tensioactifs hydrosolubles à température ambiante, contenant une chaîne grasse, saturée ou insaturée, linéaire ou ramifiée, ayant de 8 à 22 atomes de carbone.

Selon un mode de réalisation préféré des compositions aqueuses selon l'invention, la proportion en phytantriol est comprise entre 0,5 et 10 % en poids par rapport au poids total de la composition.

De préférence, le rapport pondéral entre le phytantriol et ledit agent dispersant et stabilisant tel que défini précédemment est compris entre 1 et 200, et de façon particulièrement préférée, entre 2 et 50.

Le phytantriol est un composé connu qui est notamment commercialisé sous la dénomination de "Phytantriol-63926"® par la Société Roche.

L'agent dispersant et stabilisant tel que défini précédemment est de préférence choisi parmi :
(1) les alkyl ou alcényl éthers ou esters de polyol,
(2) les amino-acides N-acylés et leurs dérivés et les peptides N-acylés par un radical alkyle ou alcényle, et leurs sels,
(3) les alkyl ou alcényl éthers ou esters sulfates, leurs dérivés et leurs sels,
(4) les alkyl ou alcényl éthers ou esters gras polyoxyéthylénés,
(5) les acides alkyl ou alcényl carboxyliques polyoxyéthylénés et leurs sels,
(6) les N-alkyl ou alcényl bétaïnes,
(7) les alkyl ou alcényl triméthylammonium et leurs sels, et
(8) leurs mélanges.

Dans les composés ci-dessus énumérés, les radicaux alkyle et alcényle ont de 8 à 22 atomes de carbone et peuvent se présenter sous forme de mélanges.

### 1- Alkyl ou alcényl éthers ou esters de polyol

Parmi ceux-ci, on peut citer notamment les alkyl ou alcényl esters de sorbitan polyoxyéthylénés par au moins 20 motifs d'oxyde d'éthylène tels que le palmitate de sorbitan 20 OE ou Polysorbate 40 commercialisé sous la dénomination de "Montanox 40 DF"® par la Société Seppic, et le laurate de sorbitan 20 OE ou Polysorbate 20 commercialisé sous la dénomination de "Tween 20"® par la Société ICI.

Dans ce groupe, on peut également citer les alkyl ou alcényl esters de polyglycérol comportant au moins 10 motifs dérivés du glycérol, oxyéthylénés ou non, tels que le polyglycéryl-10 laurate commercialisé sous la dénomination de "Decaglyn 1-L"® par la Société Nikko Chemicals.

On peut encore citer les aikyl ou alcényl éthers ou esters de mono ou polysaccharides tels que ceux dérivant du glucose, du fructose, du galactose, du maltose ou du lactose et notamment les monoesters en 1- et 6- du D-fructose, du décylglucose et du décylpolyglucose.

### 2- Amino-acides N-acylés et leurs dérivés et les peptides N-acylés par un radical alkyle ou alcényle et leurs sels

Parmi ceux-ci, on utilise de préférence ceux pour lesquels le radical alkyle ou alcényle a au moins 12 atomes de carbone.

Par amino-acides, on entend selon l'invention les α, β ou γ-amino-acides. Comme sels d'amino-acides N-acylés, on peut citer par exemple ceux de glutamate N-acylé tels que le cocoyl glutamate de monosodium, le lauroyl glutamate de monosodium, l'alcoyl C₁₄-C₂₀ glutamate de disodium (le radical alcoyle C₁₄-C₂₀ dérivant du suif hydrogéné), commercialisés respectivement sous les dénominations de "Acylglutamate CS-11"®, de "Acylglutamate LS-11"® et de "Acylglutamate HS-21"® par la Société Ajinomoto.

On peut encore citer les lysines N-acylées telles que la lauroyl lysine commercialisée sous la dénomination de "Amihope LL"® par la Société Ajinomoto.

Les dérivés d'amino-acides N-acylés et leurs sels sont de préférence les sarcosinates N-acylés tels que le lauroyl sarcosinate de sodium commercialisé sous la dénomination de "Oramix L30"® par la Société Seppic, le myristoyl sarcosinate de sodium et le palmitoyl sarcosinate de sodium commercialisés respectivement sous les dénominations de "Nikkol Sarcosinate MN"® et de "Nikkol Sarcosinate PN"®, par la Société Nikko Chemicals.

Parmi les peptides N-acylés on peut citer ceux dérivés de tout ou partie du collagène ou de la kératine tels que le lauroyl collagène de sodium et la palmitoyl kératine commercialisés sous les dénominations de "Proteol B 30"® et de "Lipacide PK"® par la Société Seppic.

### 3- Alkyl ou alcényl éthers ou esters sulfates, leurs dérivés et leurs sels

Parmi ceux-ci, on utilise de préférence ceux pour lesquels le radical alkyle ou alcényle a au moins 12 atomes de carbone.

Parmi les alkyl ou alcényl éthers sulfates, on utilise de préférence les sels d'alkyl éther sulfate et notamment le lauryléther sulfate de sodium.

Parmi les alkyl ou alcényl esters sulfates, on peut citer par exemple les esters de l'acide iséthionique ainsi que ses sels et notamment le cocoyl iséthionate de sodium commercialisé sous la dénomination de "Geropon AC 78"® par la Société Rhône Poulenc.

### 4- Alkyl ou alcényl éthers ou esters gras polyoxyéthylénés

Parmi ceux-ci, on utilise de préférence ceux pour lesquels le radical alkyle ou alcényle a au moins 12 atomes de carbone. Ceux particulièrement préférés ont au moins 20 motifs d'oxyde d'éthylène tels que par exemple le PEG-20 stéarate, le laureth-23, l'oleth-20 et le PEG-25 phytostérol.

### 5- Acides alkyl ou alcényl carboxyliques polyoxyéthylénés et leurs sels

Parmi ceux-ci, on utilise de préférence ceux comportant au moins 10 motifs d'oxyde d'éthylène tels que par exemple l'acide laureth-10 carboxylique et l'acide oleth-10 carboxylique.

### 6- N-alkyl ou alcényl bétaïnes

Parmi celles-ci, on utilise de préférence celles pour lesquelles le radical alkyle ou alcényle a au moins 12 atomes de carbone telles que par exemple la lauryl amidopropyl bétaïne et l'oléyl amidopropyl bétaïne.

### 7- Alkyl ou alcényl triméthylammonium et leurs sels

Parmi ceux-ci, on utilise de préférence ceux pour lesquels le radical alkyle ou alcényle a au moins 12 atomes de carbone. Comme sels on utilise de préférence les bromures et chlorures tels que le chlorure de cocoyltriméthylammonium et le bromure de cétyltriméthylammonium.

Les compositions cosmétiques ou dermatologiques sous forme de dispersion selon l'invention ont un pH généralement compris entre 5 et 8 et de préférence compris entre 6 et 7.

Les compositions selon l'invention sont stables et peuvent être conservées pendant 2 mois à une température comprise entre 4 et 45°C, sans présenter aucune variation d'aspect macroscopique ni microscopique, ni de couleur, ni d'odeur.

Selon un mode de réalisation particulier des compositions selon l'invention, les particules de gel cubique comprennent en outre de 0,0005 % à 5 % en poids et de préférence de 0,001 % à 2 % en poids d'un lipide amphiphile ionique non hydrosoluble.

Parmi ceux-ci, on peut notamment citer :
(i) les phopholipides tels que les phospholipides naturels comme la lécithine de soja ou d'oeuf, les phospholipides modifiés par voie chimique ou enzymatique comme la lécithine hydrogénée ou le sel de sodium de l'acide phosphatidique, et les phospholipides de synthèse comme la dipalmitoylphosphatidylcholine,
(ii) les esters phosphoriques d'alcool gras tels que le monocétyl phosphate et ses sels de sodium et de potassium commercialisé sous la dénomination de "Monafax 160"® par la Société Mona, ainsi que le dimyristyl phosphate et ses sels de sodium et de potassium commercialisé sous la dénomination de "Mexoryl SY"® par la Société Chimex,
(iii) les dérivés N-acylés de l'acide glutamique tels que le stéaroyl glutamate de monosodium commercialisé sous la dénomination de "Acylglutamate HS 11"® par la Société Ajinomoto et le mélange cocoyl-alcoyl C₁₄-C₂₀ glutamate de monosodium, le radical alcoyle C₁₄-C₂₀ dérivant du suif hydrogéné, commercialisé sous la dénomination de "Acylglutamate GS 11"® par la Société Ajinomoto,
(iv) le cétylsulfate de sodium commercialisé sous la dénomination de "Nikkol SCS"® par la Société Nikko Chemicals,
(v) le cocoyl monoglycéride sulfate de sodium commercialisé sous la dénomination de "Nikkol SGC 80 N"® par la Société Nikko Chemicals, et
(vi) les dérivés d'ammonium quaternaire tels que le chlorure de béhényltriméthylammonium, le chlorure de dilauryldiméthylammonium, le chlorure de distéaryldiméthylammonium, le méthylsulfate de 4,5-dihydro 1-méthyl 2-alcoyl C₁₄-C₂₀ 1-(2-alcoyl C₁₄-C₂₀ aminoéthyl)imidazolium, les radicaux alcoyles C₁₄-C₂₀ dérivant du suif hydrogéné, commercialisé sous la dénomination de "Rewoquat W75H"® par la Société Rewo Chemische, le méthylsulfate de dialkylhydroxyéthylméthylammonium dont les radicaux alkyles dérivent du suif, hydrogéné ou non, commercialisé sous la dénomination de "Stepanquat VP 85"® par la Société Stepan et le quaternium-82 commercialisé par la Société Seppic sous la dénomination de "Amonyl DM"®.

L'incorporation de ces lipides amphiphiles ioniques non hydrosolubles confère aux particules de gel cubique une charge superficielle entraînant une répulsion électrostatique des particules entre-elles.

Les compositions cosmétiques ou dermatologiques sous forme de dispersion de particules de gel cubique telles que définies précédemment sont obtenues par fragmentation, à l'aide d'un homogénéiseur, d'un gel cubique à base de phytantriol, d'eau, d'au moins un agent tensioactif hydrosoluble à chaîne grasse tel que défini précédemment et éventuellement de lipides amphiphiles ioniques non hydrosolubles tels que définis précédemment et/ou de principes actifs hydrophiles et lipophiles.

Les particules de gel cubique peuvent être obtenues par divers moyens mécaniques appropriés tels que par exemple par un homogénéiseur de type rotor-stator à fort gradient de cisaillement comme le "Virtis", ou par un homogénéiseur haute pression fonctionnant entre 200 et 1.800 bars environ (20 à 180 MPa).

La taille moyenne des particules de la dispersion, telle que définie précédemment, est généralement d'environ 0,05 à 1 µm, et de préférence inférieure ou égale à 0,5 µm. La granulométrie de la dispersion peut en outre être modulée par la nature et la concentration de l'agent tensioactif hydrosoluble à chaîne grasse utilisé.

Il est possible d'incorporer dans les particules de gel cubique des dispersions telles que définies précédemment, divers types de composés actifs. En particulier lesdites particules peuvent contenir un principe actif hydrophile ou un principe actif lipophile.

Bien entendu, grâce à la structure particulière des particules de gel cubique, il est possible d'incorporer dans celles-ci à la fois des principes actifs hydrophiles et des principes actifs lipophiles même si ceux-ci présentent une certaine incompatibilité.

Parmi les différents principes actifs pouvant être incorporés, on peut notamment citer :
1) les agents antioxydants ou anti-radicaux libres tels que :
   - les protéines et les enzymes telles que la superoxyde dismutase (SOD), la lactoperoxydase et la lactoferrine,
   - les peptides et leurs dérivés tels que la taurine et la carnosine,
   - les séquestrants tels que l'acide phytique et les dérivés polyphosphoniques,
   - les flavonoïdes tels que la rutine et l'α-glycosyl rutine,
   - la chlorophylline,
   - l'éthoxyquine,
   - la guanosine,
   - les tocophérols, notamment les α,β ou γ tocophérols et en particulier le d-α-tocophérol commercialisé sous la dénomination de "Covitol F 1300"® par la Société Henkel ainsi que l'acétate de tocophérol,
   - le palmitate d'ascorbyle, et
   - le β-carotène,
2) les agents hydratants ou humectants tels que :
   - l'acide hyaluronique et son sel de sodium,
   - le β-glycérophosphate,
   - le glycérol, et
   - le sorbitol,
3) les filtres UV tels que :
   les produits commercialisés sous les dénominations de "Eusolex 232"® par la Société Merck, de "Parsol 1789"® et de "Parsol MCX"® par la Société Givaudan-Roure, de "Mexoryl SX"® par la Société Chimex et de "Uvinul T150"® par la Société BASF,
4) les kératolytiques tels que :
   - les enzymes protéolytiques telles que la subtilisine, la trypsine, l'α-chymotrypsine et la papaïne,
   - l'acide salicylique et ses dérivés tels que l'acide n-dodécanoyl-5 salicylique, et
   - l'acide rétinoïque,
5) les accélérateurs de bronzage tels que :
   - la caféine, et
   - les dérivés de tyrosine tels que le tyrosinate de glucose et le sel disodique de la N-L-malyl tyrosine,
6) les dépigmentants tels que :
   - l'acide kojique,
   - l'acide glycolique,
   - la vitamine C et notamment l'ascorbyle phosphate de magnésium, et
   - l'arbutine et ses dérivés,
7) les colorants naturels tels que :
   - les matières colorantes extraites de végétaux comme la chlorophylline et le β-carotène ou extraites d'animaux comme le carmin de cochenille, et
   - le caramel,
8) les auto-bronzants tels que :
   - la dihydroxyacétone, et
   - les indoles,
9) les liporégulateurs tels que :
   - le γ-orizanol,
   - l'extrait de *Centella asiatica* contenant de la génine et de l'acide asiatique,
   - la caféine, et
   - la théophylline,
10) les agents anti-vieillissement et anti-rides tels que :
   - les hydroxyacides comme l'acide glycolique,
   - l'acide n-octanoyl salicylique,
   - le rétinol et ses dérivés comme l'acétate, le palmitate et le propionate de rétinol, et
   - les rétinoïdes,
11) les agents anti-inflammatoires et cicatrisants tels que :
   - l'acide 18 β-glycyrrhétinique et ses sels comme notamment son sel d'ammonium,
   - l'α-bisabolol,
   - les corticoïdes, et
   - l'extrait de *Centella asiatica*,
12) les antibactériens et antifongiques tels que :
   - le chlorure de benzalkonium,
   - la chlorhexidine,
   - l'hexetidine, et
   - l'hexamidine,
13) les insectifuges tels que :
   - les diéthyl et diméthyltoluamides,
14) les déodorants tels que :
   - l'hexachlorophène, et
   - le triclosan produit commercialisé sous la dénomination de "Irgasan DP 300"® par la Société Ciba-Geigy,
15) les antipelliculaires tels que :
   - l'octopirox, et
   - les dérivés de pyridinethione tels que ceux commercialisés sous les dénominations de "Omadine"® par la Société Olin,
16) les agents anti-chute des cheveux tels que :
   - le nicotinate de méthyle ou d'hexyle, et
   - le minoxidil,
17) les colorants capillaires tels que :
   - les bases et les coupleurs d'oxydation,
   - les colorants directs, et
   - les colorants auto-oxydables,
18) les agents réducteurs pour permanentes tels que :
   - l'acide thioglycolique,
   - la cystéine,
   - la cystéamine,
   - la N-acétyl cystéine,
   - la N-acétyl cystéamine, et
   - le thioglycolate de glycérol,
19) les agents conditionneurs pour peau et cheveux tels que :
   - les polymères cationiques et les cations.

Les compositions sous forme de dispersion selon l'invention peuvent donc comprendre soit des particules contenant des principes actifs hydrophiles, soit des particules contenant des principes actifs lipophiles, soit des particules contenant à la fois des principes actifs hydrophiles et lipophiles tels que par exemple des filtres UV hydrophiles et lipophiles, ou un mélange de ces différentes particules.

Il est également possible d'incorporer dans la phase aqueuse continue de la dispersion différents composés cosmétiquement ou dermatologiquement acceptables tels que des principes actifs hydrophiles comme des agents hydratants ou additifs conventionnels.

Parmi ces derniers, on peut citer notamment des agents conservateurs, des parfums, des pigments (TiO₂), des matières colorantes, des charges, des gélifiants, etc.

Les compositions selon l'invention peuvent également comprendre, outre les particules dispersées de gel cubique, des liposomes contenant éventuellement des principes actifs.

La présence de phytantriol dans les compositions selon l'invention leur confère un bon pouvoir hydratant.

La présente invention a donc également pour objet l'utilisation d'une composition telle que définie précédemment pour hydrater la peau par application topique.

La présente invention a en outre pour objet un procédé d'hydratation de la peau comprenant l'application sur la peau d'une composition telle que définie précédemment.

On va maintenant donner à titre d'illustration des exemples de préparation de dispersions aqueuses selon l'invention à usage cosmétique et dermatologique.

### EXEMPLE 1 : Composition cosmétique sous forme d'une dispersion de particules de gel cubique de phytantriol stabilisée par du polysorbate 40.

A un gel cubique obtenu par mélange de 1 g de phytantriol et de 0,43 g d'eau, on ajoute 18,57 g d'une solution aqueuse contenant 2 % de polysorbate 40 commercialisé sous la dénomination de "Montanox 40 DF"® par la Société Seppic. Le mélange ainsi obtenu est alors prédispersé puis homogénéisé à température ambiante à l'aide d'un homogénéiseur de type "Virtis" à 35000 tr/min pendant 5 minutes, cette agitation étant répétée 4 fois.

La composition sous forme de dispersion ainsi obtenue est homogène et stable. Conservée entre 4°C et 45°C pendant 2 mois, elle ne présente en effet ni variation de couleur, ni d'odeur, ni l'apparition de cristaux.

La taille moyenne des particules, mesurée à l'aide d'un granulomètre laser CBI 90 de la Société Brookhaven Instruments Corporation, est environ de 0,48 µm.

Appliquée sur la peau, la composition sous forme de dispersion ainsi obtenue s'étale facilement et présente des qualités sensorielles satisfaisantes ainsi qu'un bon pouvoir hydratant.

### Evaluation de l'effet hydratant du phytantriol

On a évalué l'effet hydratant du phytantriol selon la méthode décrite dans "Impedance methods for studying skin moisturization", J.L. Lévèque et al., Journ. Soc. Cosmet. Chem., 34, 419-428, Dec.1983.

Selon cette méthode, l'augmentation de la teneur en eau de la peau est corrélée à une augmentation de la conductance électrique de celle-ci et permet donc une mesure indirecte de l'hydratation de la peau.

On applique sur les avant-bras de 12 personnes présentant des peaux dites "sèches", l'une des compositions suivantes à raison de 2mg/cm² :

### Composition A (selon l'invention) :

Composition de l'exemple 1, c'est-à-dire contenant 5 % en poids de phytantriol et 1,7 % en poids de polysorbate 40 par rapport au poids total de la composition,

### Composition B (comparatif) :

Dispersion aqueuse de particules de gel cubique préparée selon le mode opératoire décrit dans la demande de brevet WO-93.06921, contenant 5 % de monooléine et 1,7 % d'un polymère bloc amphiphile commercialisé sous la dénomination de "Pluronic F127"® par la Société BASF, les pourcentages étant exprimés en poids par rapport au poids total de la composition,

### Composition C (comparatif) :

Solution aqueuse contenant 1,7 % en poids de polysorbate 40 par rapport au poids total de la composition .

Une heure après l'application, on mesure la conductance électrique à haute fréquence (10 MHz) à l'aide d'un appareil de type "Dermodiag" selon le principe décrit dans BF 73.10935 et BF 75.18905.

Les résultats sont exprimés en variation d'hydratation par rapport à la peau nue et sont présentés dans le tableau suivant :

| Composition | A | B | C | Peau nue |
|---|---|---|---|---|
| Variation d'hydratation après 1 h d'application | 14±7 | 6±4* | 3±6* | -1±4* |

| | | | | |
|---|---|---|---|---|
| *Valeurs qui ne sont pas significativement différentes selon le test statistique intitulé "Analyse des Variances" ou test ANOVA | | | | |

Il apparaît ainsi que seule la composition A sous forme d'une dispersion de particules de gel cubique de phytantriol et de polysorbate 40 présente un effet hydratant sur la peau, effet qui ne peut être obtenu à l'aide des compositions B et C telles que définies ci-dessus. Dans la mesure où aucun effet hydratant n'est observé après application de la composition C, l'effet hydratant observé après application de la composition A résulte bien de la présence du phytantriol.

### EXEMPLE 2 : Composition cosmétique sous forme d'une dispersion de particules de gel cubique à base de phytantriol et d'une lécithine stabilisée par du polysorbate 40.

A un gel cubique obtenu par mélange de 0,3 g d'eau et de 0,7 g d'un mélange en un rapport de 70/30 en poids de phytantriol et d'une lécithine commercialisée sous la dénomination de "Epikuron 145 V"® par la Société Lucas Meyer, on ajoute 19 g d'une solution aqueuse contenant 1 % de polysorbate 40. Le mélange est alors homogénéisé à température ambiante à l'aide d'un homogénéiseur de type "Heidolph" (Diax 600) muni d'une tête de dispersion 18 G, à 25000 tr/min pendant 15 minutes.

La composition ainsi obtenue est homogène et stable.

La taille moyenne des particules est d'environ 0,17 µm.

### EXEMPLE 3 : Composition cosmétique sous forme d'une dispersion de particules de gel cubique de phytantriol contenant 0,05 % de superoxyde dismutase (SOD) stabilisée par du polyglycéryl-10 laurate.

A 1 g de gel cubique obtenu par mélange à température ambiante de 0,7 g de phytantriol et de 0,3 g d'une solution aqueuse contenant 10 mg de SOD, on ajoute après équilibre 19 g d'une solution aqueuse contenant 2 % de polyglycéryl-10 laurate commercialisé sous la dénomination de "Decaglyn 1-L"® par la Société Nikko Chemicals. Le mélange ainsi obtenu est homogénéisé à température ambiante à l'aide d'un homogénéiseur haute pression de type "Soavi" par 4 passages à 600 bars.

La composition ainsi obtenue est homogène et stable. Appliquée sur la peau, elle confère un effet protecteur contre les agressions radicalaires qui accélèrent le vieillissement de la peau. Cette composition possède donc un excellent effet anti-vieillissement.

La taille moyenne des particules est d'environ 0,14 µm.

### EXEMPLE 4 : Composition cosmétique sous forme d'une dispersion aqueuse de particules de gel cubique de phytantriol contenant du d-α-tocophérol et de la superoxyde dismutase (SOD) stabilisée par du polysorbate 40.

A un gel cubique obtenu par mélange de 0,7 g d'un mélange de phytantriol et d'α-tocophérol (97/3) et de 0,3 g d'une solution aqueuse contenant 10 mg de SOD, on ajoute à température ambiante, 19 g d'une solution aqueuse contenant 1 % de polysorbate 40. Le mélange ainsi obtenu est homogénéisé à température ambiante à l'aide d'un homogénéiseur de type "Heidolph" (Diax 600) muni d'une tête de dispersion 18G, à 25000 tr/min pendant 15 minutes. La composition obtenue est stable et homogène et contient à la fois des agents anti-radicaux libres de type hydrophile et lipophile.

La taille moyenne des particules de la composition est d'environ 0,26 µm.

### EXEMPLE 5 : Composition cosmétique sous forme d'une dispersion de particules de gel cubique de phytantriol contenant du "Parsol MCX"® et du "Mexoryl SX"® stabilisée par du polysorbate 40.

A 1 g du gel cubique obtenu par mélange de 7 g d'un mélange en un rapport de 97/3 en poids de phytantriol et d'un filtre solaire lipophile commercialisé sous la dénomination de "Parsol MCX"® par la Société Givaudan-Roure et de 3 g d'une solution aqueuse contenant 0,455 g d'un filtre solaire hydrophile commercialisé sous la dénomination de "Mexoryl SX"® par la Société Chimex, on ajoute 19 g d'une solution aqueuse contenant 1 % de polysorbate 40. Le mélange est homogénéisé à température ambiante à l'aide d'un homogénéiseur de type "Heidolph" (Diax 600) muni d'une tête de dispersion 18G à 25000 tr/min pendant 15 minutes.

La composition ainsi obtenue est stable et homogène comprenant une association de filtres solaires couvrant un large spectre de radiations et constitue donc un bon produit anti-solaire.

La taille moyenne des particules du gel cubique est d'environ 0,25 µm.

### EXEMPLE 6 : Composition cosmétique sous forme d'une dispersion de particules de gel cubique de phytantriol et de monocétylphosphate contenant de l'éthoxyquine, de la lactoperoxydase et de la lactoferrine, stabilisée par du polysorbate 40

A 7,1 g du gel cubique obtenu par mélange des composés suivants :
- Phytantriol 4,85 g
- Monocétyl phosphate commercialisé sous la dénomination de "Monofax 160"® par la Société Mona 0,1 g
- Ethoxyquine commercialisée sous la dénomination de "Raluquin"® par la Société Raschig 0,05 g
- Solution aqueuse contenant 0,05 g de lactoperoxydase et 0,04 g de lactoferrine commercialisées par la Société Bioserae 2,1 g
on ajoute après équilibre, à température ambiante, 92,9 g d'une solution aqueuse contenant 1,4g de polysorbate 40 et 0,0125 g de soude.

Le mélange obtenu est homogénéisé à température ambiante à l'aide d'un homogénéiseur de type "Virtis" à 35000 tr/min pendant 3 minutes, cette agitation étant répétée 2 fois.

La composition ainsi obtenue est homogène et stable et constitue un excellent produit anti-vieillissement.

La taille moyenne des particules est d'environ 0,20 µm.

## Revendications

1. Composition cosmétique ou dermatologique, à usage topique, sous forme d'une dispersion, aqueuse et stable, de particules de gel cubique, caractérisée par le fait qu'elle comprend :
(a) de 0,1 à 15 % en poids de 3,7,11,15-tétraméthyl 1,2,3-hexadecanetriol ou phytantriol par rapport au poids total de la composition, et
(b) de 0,1 à 3 % en poids d'un agent dispersant et stabilisant par rapport au poids total de la composition, ledit agent étant choisi parmi les agents tensioactifs hydrosolubles à température ambiante, contenant une chaîne grasse, saturée ou insaturée, linéaire ou ramifiée, ayant de 8 à 22 atomes de carbone.

2. Composition selon la revendication 1, caractérisée par le fait que la proportion en phytantriol est comprise entre 0,5 et 10 % en poids par rapport au poids total de la composition.

3. Composition selon l'une quelconque des revendications précédentes, caractérisée par le fait que le rapport en poids entre le phytantriol et ledit agent dispersant et stabilisant est compris entre 1 et 200.

4. Composition selon la revendication 1 ou 2, caractérisée par le fait que le rapport en poids entre le phytantriol et ledit agent dispersant et stabilisant est compris entre 2 et 50.

5. Composition selon l'une quelconque des revendications précédentes, caractérisée par le fait que ledit agent stabilisant est choisi parmi :
(1) les alkyl ou alcényl éthers ou esters de polyol,
(2) les amino-acides N-acylés et leurs dérivés et les peptides N-acylés par un radical alkyle ou alcényle, et leurs sels,
(3) les alkyl ou alcényl éthers ou esters sulfates, leurs dérivés et leurs sels,
(4) les alkyl ou alcényl éthers ou esters gras polyoxyéthylénés,
(5) les acides alkyl ou alcényl carboxyliques polyoxyéthylénés et leurs sels,
(6) les N-alkyl ou alcényl bétaïnes,
(7) les alkyl ou alcényl triméthylammonium et leurs sels, et
(8) leurs mélanges.

6. Composition selon la revendication 5, caractérisée par le fait que lesdits alkyl ou alcényl éthers ou esters de polyol sont choisis parmi les alkyl ou alcényl esters de sorbitan polyoxyéthylénés par au moins 20 motifs d'oxyde d'éthylène, les alkyl ou alcényl esters de polyglycérol comportant au moins 10 motifs dérivés du glycérol, oxyéthylénés ou non, et les alkyl ou alcényl éthers ou esters de mono ou polysaccharides.

7. Composition selon la revendication 6, caractérisée par le fait que lesdits alkyl ou alcényl éthers ou esters de polyol sont choisis parmi le palmitate de sorbitan 20 OE, le laurate de sorbitan 20 OE, le polyglycéryl-10 laurate, les monoesters en 1- et 6- du D fructose, du décylglucose et du décylpolyglucose.

8. Composition selon la revendication 5, caractérisée par le fait que lesdits amino-acides N-acylés et leurs dérivés et lesdits peptides N-acylés et leurs sels sont choisis parmi les glutamates N-acylés, les lysines N-acylées, les aminopropionates N-acylés, les sarcosinates N-acylés, et tout ou partie du collagène N-acylé ou de la kératine N-acylée.

9. Composition selon la revendication 8, caractérisée par le fait que lesdits amino-acides N-acylés et leurs dérivés et lesdits peptides N-acylés et leurs sels sont choisis parmi le cocoyl glutamate de monosodium, le lauroyl glutamate de monosodium, l'alcoyl C₁₄-C₂₀ glutamate de disodium, le radical alcoyle dérivant du suif hydrogéné, la lauroyl lysine, le lauroyl sarcosinate de sodium, le myristoyl sarcosinate de sodium, le palmitoyl sarcosinate de sodium, le lauroyl collagène de sodium et la palmitoyl kératine.

10. Composition selon la revendication 5, caractérisée par le fait que lesdits sels d'alkyl ou alcényl éthers ou esters sulfate sont choisis parmi le lauryléther sulfate de sodium et le cocoyl iséthionate de sodium.

11. Composition selon la revendication 5, caractérisée par le fait que lesdits alkyl ou alcényl esters ou éthers gras polyoxyéthylénés comportent au moins 20 motifs d'oxyde d'éthylène.

12. Composition selon la revendication 11, caractérisée par le fait que lesdits alkyl ou alcényl esters ou éthers gras polyoxyéthylénés sont choisis parmi le PEG-20 stéarate, le laureth-23, l'oleth-20 et le PEG-25 phytostérol.

13. Composition selon la revendication 5, caractérisée par le fait que lesdits acides alkyl ou alcényl carboxyliques polyoxyéthylénés et leurs sels comportent au moins 10 motifs d'oxyde d'éthylène.

14. Composition selon la revendication 13, caractérisée par le fait que lesdits acides alkyl ou alcényl carboxyliques polyoxyéthylénés sont choisis parmi l'acide laureth-10 carboxylique et l'acide oleth-10 carboxylique.

15. Composition selon la revendication 5, caractérisée par le fait que lesdites N-alkyl ou alcényl bétaïnes sont choisies parmi la lauryl amidopropylbétaïne et l'oléyl amidopropylbétaïne.

16. Composition selon la revendication 5, caractérisée par le fait que lesdits sels d'alkyl ou alcényl triméthylammonium sont choisis parmi le chlorure de cocoyltriméthylammonium et le bromure de cétyltriméthylammonium.

17. Composition selon l'une quelconque des revendications précédentes, caractérisée par le fait que lesdites particules de gel cubique contiennent en outre de 0,0005 % à 5 % en poids d'au moins un lipide amphiphile ionique non hydrosoluble par rapport au poids total de la composition .

18. Composition selon la revendication 17, caractérisée par le fait que la proportion en lipide amphiphile ionique non hydrosoluble est de 0,001 % à 2 % en poids par rapport au poids total de la composition.

19. Composition selon l'une quelconque des revendications 17 et 18, caractérisée par le fait que ledit lipide amphiphile ionique non hydrosoluble est choisi parmi :
(i) les phospholipides,
(ii) les esters phosphoriques d'alcool gras,
(iii) les dérivés N-acylés de l'acide glutamique non hydrosolubles,
(iv) le cétylsulfate de sodium,
(v) le cocoyl monoglycéride sulfate de sodium, et
(vi) les dérivés d'ammonium quaternaire non hydrosolubles.

20. Composition selon la revendication 19, caractérisée par le fait que ledit phospholipide est choisi parmi la lécithine de soja, la lécithine d'oeuf, la lécithine hydrogénée, le sel de sodium de l'acide phosphatidique et la dipalmitoylphosphatidylcholine.

21. Composition selon la revendication 19, caractérisée par le fait que ledit ester phosphorique d'alcool gras est choisi parmi le monocétyl phosphate et le dimyristyl phosphate et leurs sels de sodium et de potassium.

22. Composition selon la revendication 19, caractérisée par le fait que ledit dérivé N-acylé de l'acide glutamique non hydrosoluble est choisi parmi le stéroyl glutamate de monosodium et le mélange de cocoyl-alcoyl C₁₄-C₂₀ glutamate de monosodium, le radical alcoyle C₁₄-C₂₀ dérivant du suif hydrogéné.

23. Composition selon la revendication 19, caractérisée par le fait que ledit dérivé d'ammonium quaternaire non hydrosoluble est choisi parmi le chlorure de béhényltriméthylammonium, le chlorure de dilauryldiméthylammonium, le chlorure de distéaryldiméthylammonium, le méthylsulfate de 4,5-dihydro 1-méthyl 2-alcoyl C₁₄-C₂₀ 1-(2-alcoyl C₁₄-C₂₀ aminoéthyl) imidazolium dont les radicaux alcoyles C₁₄-C₂₀ dérivent du suif hydrogéné, le méthylsulfate de dialkylhydroxyéthylméthylammonium dont les radicaux alkyles dérivent du suif, hydrogéné ou non, et le quaternium-82.

24. Composition selon l'une quelconque des revendications précédentes, caractérisée par le fait que lesdites particules ont une taille moyenne d'environ 0,05 µm à 1µm.

25. Composition selon l'une quelconque des revendications précédentes, caractérisée par le fait que lesdites particules ont une taille moyenne inférieure ou égale à 0,5 µm.

26. Composition selon l'une quelconque des revendications précédentes, caractérisée par le fait que lesdites particules contiennent au moins un principe actif hydrophile.

27. Composition selon l'une quelconque des revendications précédentes, caractérisée par le fait que lesdites particules contiennent au moins un principe actif lipophile.

28. Composition selon l'une quelconque des revendications précédentes, caractérisée par le fait que lesdites particules contiennent à la fois au moins un principe actif hydrophile et au moins un principe actif lipophile.

29. Composition selon l'une quelconque des revendications précédentes, caractérisée par le fait qu'elle contient en outre dans la phase aqueuse au moins un composé cosmétiquement ou dermatologiquement acceptable choisi parmi un principe actif hydropile, un agent conservateur, un parfum, un pigment, une matière colorante, une charge et un gélifiant.

30. Composition selon l'une quelconque des revendications précédentes, caractérisée par le fait qu'elle comprend en outre des liposomes.

31. Utilisation d'une composition telle que définie selon l'une quelconque des revendications précédentes pour hydrater la peau par application topique.

32. Procédé cosmétique d'hydratation de la peau, caractérisé par le fait qu'il consiste à appliquer sur la peau une composition telle que définie selon l'une quelconque des revendications 1 à 30, contenant éventuellement un principe actif cosmétique.

## Patentansprüche

1. Kosmetische oder dermatologische Zubereitung zur topischen Verwendung in Form einer wässrigen und stabilen Dispersion von Partikeln eines kubischen Gels, dadurch gekennzeichnet, dass diese umfasst:
(a) 0,1 bis 15 Gew.-%, bezogen auf das Gesamtgewicht der Zubereitung, 3,7,11,15-Tetramethyl-1,2,3-hexadecantriol oder Phytantriol und
(b) 0,1 bis 3 Gew.-%, bezogen auf das Gesamtgewicht der Zubereitung, eines Dispergier- und Stabilisierungsmittels, wobei das Mittel ausgewählt wird unter den grenzflächenaktiven, bei Raumtemperatur wasserlöslichen Mitteln, die eine gesättigte oder ungesättigte, lineare oder verzweigte Fettsäurekette mit 8 bis 22 Kohlenstoffatomen aufweisen.

2. Zubereitung nach Anspruch 1, dadurch gekennzeichnet, dass der Phytantriol-Anteil 0,5 bis 10 Gew.-% beträgt, bezogen auf das Gesamtgewicht der Zubereitung.

3. Zubereitung nach einem der vorstehenden Ansprüche, dadurch gekennzeichnet, dass das Gewichtsverhältnis von Phytantriol zu dem Dispergier- und Stabilisierungsmittel 1 bis 200 beträgt.

4. Zubereitung nach Anspruch 1 oder 2, dadurch gekennzeichnet, dass das Gewichtsverhältnis von Phytantriol zu dem Dispergier- und Stabilisierungsmittel 2 bis 50 beträgt.

5. Zubereitung nach einem der vorstehenden Ansprüche, dadurch gekennzeichnet, dass das Stabilisierungsmittel ausgewählt wird unter:
(1) Polyolalkyl- oder -alkenylethern oder -estern,
(2) N-acylierten Aminosäuren und deren Derivaten und mit einem Alkyl- oder Alkenylrest N-acylierten Peptiden und deren Salzen,
(3) Alkyl- oder Alkenylether- oder -estersulfaten, deren Derivaten und deren Salzen,
(4) polyoxyethylenierten Fettsäurealkyl- oder -alkenylethern oder -estern,
(5) polyoxyethylenierten Alkyl- oder Alkenylcarbonsäuren und deren Salzen,
(6) N-Alkyl- oder -Alkenylbetainen,
(7) Alkyl- oder Alkenyltrimethylammonium und dessen Salzen und
(8) deren Gemischen.

6. Zubereitung nach Anspruch 5, dadurch gekennzeichnet, dass die Polyolalkyl- oder -alkenylether oder -ester ausgewählt werden unter den polyoxyethylenierten Sorbitanalkyl- oder -alkenylestern mit mindestens 20 Ethylenoxideinheiten, den Polyglycerinalkyl- oder -alkenylestern mit mindestens 10 von Glycerin abgeleiteten Einheiten, oxyethyleniert oder nicht, und den Mono- oder Polysaccharidalkyl- oder -alkenylethern oder -estern.

7. Zubereitung nach Anspruch 6, dadurch gekennzeichnet, dass die Polyolalkyl- oder -alkenylether oder -ester ausgewählt werden unter Sorbitanpalmitat 20 EO, Sorbitanlaurat 20 EO, Polyglyceryl-10-laurat, den 1- und 6-Monoestern der D-Fructose, Decylglucose und Decylpolyglucose.

8. Zubereitung nach Anspruch 5, dadurch gekennzeichnet, dass die N-acylierten Aminosäuren und deren Derivate und die N-acylierten Peptide und deren Salze ausgewählt werden unter den N-acylierten Glutamaten, den N-acylierten Lysinen, den N-acylierten Aminopropionaten, den N-acylierten Sarcosinaten und dem gesamten oder Teilen von N-acyliertem Kollagen oder N-acyliertem Keratin.

9. Zubereitung nach Anspruch 8, dadurch gekennzeichnet, dass die N-acylierten Aminosäuren und deren Derivate und die N-acylierten Peptide und deren Salze ausgewählt werden unter Mononatriumcocoylglutamat, Mononatriumlauroylglutamat, Dinatrium-C₁₄-C₂₀-alkoylglutamat mit von hydriertem Talk abgeleitetem Alkoylrest, Lauroyllysin, Natriumlauroylsarcosinat, Natriummyristoylsarcosinat, Natriumpalmitoylsarcosinat, Natriumlauroylkollagen und Palmitoylkeratin.

10. Zubereitung nach Anspruch 5, dadurch gekennzeichnet, dass die Alkyl- oder Alkenylether- oder -estersulfatsalze ausgewählt werden unter Natriumlaurylethersulfat und Natriumcocoylisethionat.

11. Zubereitung nach Anspruch 5, dadurch gekennzeichnet, dass die polyoxyethylenierten Fettsäurealkyl- oder -alkenylester oder -ether mindestens 20 Ethylenoxideinheiten aufweisen.

12. Zubereitung nach Anspruch 11, dadurch gekennzeichnet, dass die polyoxyethylenierten Fettsäurealkyl- oder -alkenylester oder -ether ausgewählt werden unter PEG-20-Stearat, Laureth-23, Oleth-20 und PEG-25-Phytosterol.

13. Zubereitung nach Anspruch 5, dadurch gekennzeichnet, dass die polyoxyethylenierten Alkyl- oder Alkenylcarbonsäuren und deren Salze mindestens 10 Ethylenoxideinheiten aufweisen.

14. Zubereitung nach Anspruch 13, dadurch gekennzeichnet, dass die polyoxyethylenierten Alkyl- oder Alkenylcarbonsäuren ausgewählt werden unter Laureth-10-carbonsäure und Oleth-10-carbonsäure.

15. Zubereitung nach Anspruch 5, dadurch gekennzeichnet, dass die N-Alkyl oder -Alkenylbetaine ausgewählt werden unter Laurylamidopropylbetain und Oleylamidopropylbetain.

16. Zubereitung nach Anspruch 5, dadurch gekennzeichnet, dass die Alkyl- oder Alkenyltrimethylammoniumsalze ausgewählt werden unter Cocoyltrimethylammoniumchlorid und Cetyltrimethylammoniumbromid.

17. Zubereitung nach einem der vorstehenden Ansprüche, dadurch gekennzeichnet, dass die Partikel des kubischen Gels außerdem 0,0005 bis 5 Gew.-%, bezogen auf das Gesamtgewicht der Zubereitung, mindestens eines ionischen, nicht wasserlöslichen Lipidamphiphils enthalten.

18. Zubereitung nach Anspruch 17, dadurch gekennzeichnet, dass der Anteil des ionischen, nicht wasserlöslichen Lipidamphiphils 0,001 bis 2 Gew.-%, bezogen auf das Gesamtgewicht der Zubereitung, beträgt.

19. Zubereitung nach einem der Ansprüche 17 und 18, dadurch gekennzeichnet, dass das ionische, nicht wasserlösliche Lipidamphiphil ausgewählt wird unter:
(i) den Phospholipiden,
(ii) den Fettalkoholphosphorsäureestern,
(iii) den nicht wasserlöslichen N-acylierten Derivaten der Glutaminsäure,
(iv) Natriumcetylsulfat,
(v) Natriumcocoylmonoglyceridsulfat und
(vi) den nicht wasserlöslichen quaternären Ammoniumderivaten.

20. Zubereitung nach Anspruch 19, dadurch gekennzeichnet, dass das Phospholipid ausgewählt wird unter Sojalecithin, Eilecithin, hydriertem Lecithin, dem Natriumsalz der Phosphatidsäure und Dipalmitoylphosphatidylcholin.

21. Zubereitung nach Anspruch 19, dadurch gekennzeichnet, dass der Fettalkoholphosphorsäureester ausgewählt wird unter Monocetylphosphat und Dimyristylphosphat und deren Natrium- und Kaliumsalzen.

22. Zubereitung nach Anspruch 19, dadurch gekennzeichnet, dass das nicht wasserlösliche N-acylierte Derivat der Glutaminsäure ausgewählt wird unter Mononatriumsteroylglutamat und dem Mononatrium-cocoyl-C₁₄-C₂₀-alkoylglutamat-Gemisch mit von hydriertem Talg abgeleitetem C₁₄-C₂₀-Alkoylrest.

23. Zubereitung nach Anspruch 19, dadurch gekennzeichnet, dass das nicht wasserlösliche quaternäre Ammoniumderivat ausgewählt wird unter Behenyltrimethylammoniumchlorid, Dilauryldimethylammoniumchlorid, Distearyldimethylammoniumchlorid, 4,5-Dihydro-1-methyl-2-C₁₄-C₂₀-alkoyl-1-(2-C₁₄-C₂₀-alkoylaminoethyl)-imidazoliummethylsulfat, dessen C₁₄-C₂₀-Alkoylreste sich von hydriertem Talg ableiten, Dialkylhydroxyethylmethylammoniummethylsulfat, dessen Alkylreste sich von gegebenenfalls hydriertem Talg ableiten, und Quaternium-82.

24. Zubereitung nach einem der vorstehenden Ansprüche, dadurch gekennzeichnet, dass die Partikel eine durchschnittliche Größe von ungefähr 0,05 µm bis 1 µm aufweisen.

25. Zubereitung nach einem der vorstehenden Ansprüche, dadurch gekennzeichnet, dass die Partikel eine durchschnittliche Größe von weniger als oder gleich 0,5 µm aufweisen.

26. Zubereitung nach einem der vorstehenden Ansprüche, dadurch gekennzeichnet, dass die Partikel mindestens einen hydrophilen Wirkstoff enthalten.

27. Zubereitung nach einem der vorstehenden Ansprüche, dadurch gekennzeichnet, dass die Partikel mindestens einen lipophilen Wirkstoff enthalten.

28. Zubereitung nach einem der vorstehenden Ansprüche, dadurch gekennzeichnet, dass die Partikel gleichzeitig mindestens einen hydrophilen Wirkstoff und mindestens einen liphophilen Wirkstoff enthalten.

29. Zubereitung nach einem der vorstehenden Ansprüche, dadurch gekennzeichnet, dass diese in der wässrigen Phase außerdem mindestens eine kosmetisch und dermatologisch annehmbare Verbindung enthält, ausgewählt unter einem hydrophilen Wirkstoff, einem Konservierungsmittel, einem Parfüm, einem Pigment, einem Farbstoff, einem Füllstoff und einem Geliermittel.

30. Zubereitung nach einem der vorstehenden Ansprüche, dadurch gekennzeichnet, dass diese außerdem Liposome umfasst.

31. Verwendung einer Zubereitung, die gemäß einem der vorstehenden Ansprüche definiert ist, zum Hydratisieren der Haut durch topische Anwendung.

32. Kosmetisches Verfahren der Hauthydratation, dadurch gekennzeichnet, dass auf die Haut eine Zubereitung, die gemäß einem der Ansprüche 1 bis 30 definiert ist und gegebenenfalls einen kosmetischen Wirkstoff enthält, angewendet wird.

## Claims

1. Cosmetic or dermatological composition for topical use, in the form of an aqueous and stable dispersion of cubic gel particles, characterized in that it comprises:
(a) from 0.1 to 15% by weight of 3,7,11,15-tetramethyl-1,2,3-hexadecanetriol or phytanetriol relative to the total weight of the composition, and
(b)from 0.1 to 3% by weight of a dispersing and stabilizing agent relative to the total weight of the composition, the said agent being chosen from surface-active agents that are water-soluble at room temperature, containing a linear or branched, saturated or unsaturated fatty chain having from 8 to 22 carbon atoms.

2. Composition according to Claim 1, characterized in that the proportion of phytanetriol is between 0.5 and 10% by weight relative to the total weight of the composition.

3. Composition according to either of the preceding claims, characterized in that the weight ratio between the phytanetriol and the said dispersing and stabilizing agent is between 1 and 200.

4. Composition according to Claim 1 or 2, characterized in that the weight ratio between the phytanetriol and the said dispersing and stabilizing agent is between 2 and 50.

5. Composition according to any one of the preceding claims, characterized in that the said stabilizing agent is chosen from:
(1)polyol alkyl or alkenyl ethers or esters,
(2)N-acylated amino acids and derivatives thereof and peptides N-acylated with an alkyl or alkenyl radical, and salts thereof,
(3)alkyl or alkenyl ether or ester sulphates, and the derivatives and salts thereof,
(4)polyoxyethylenated alkyl or alkenyl fatty ethers or esters,
(5)polyoxyethylenated alkyl or alkenyl carboxylic acids and salts thereof,
(6)N-alkyl or N-alkenyl betaines,
(7)alkyltrimethylammonium or alkenyltrimethylammonium and salts thereof, and
(8)mixtures thereof.

6. Composition according to Claim 5, characterized in that the said polyol alkyl or alkenyl ethers or esters are chosen from alkyl or alkenyl esters of sorbitan polyoxyethylenated with at least 20 units of ethylene oxide, polyglyceryl alkyl or alkenyl esters containing at least 10 units derived from glycerol, which may or may not be oxyethylenated, and alkyl or alkenyl mono- or poly-saccharide ethers or esters.

7. Composition according to Claim 6, characterized in that the said polyol alkyl or alkenyl ethers or esters are chosen from sorbitan palmitate 20 EO, sorbitan laurate 20 EO, polyglyceryl-10 laurate, and the monoesters in positions -1 and -6 of D-fructose, of decylglucose and of decylpolyglucose.

8. Composition according to Claim 5, characterized in that the said N-acylated amino acids and derivatives thereof and the said N-acylated peptides and salts thereof are chosen from N-acylated glutamates, N-acylated lysines, N-acylated aminopropionates, N-acylated sarcosinates, and all or part of N-acylated collagen or N-acylated keratin.

9. Composition according to Claim 8, characterized in that the said N-acylated amino acids and derivatives thereof and the said N-acylated peptides and salts thereof are chosen from monosodium cocoyl glutamate, monosodium lauroyl glutamate, disodium C₁₄-C₂₀ alkoyl glutamate, the alkoyl radical being derived from hydrogenated tallow, lauroyl lysine, sodium lauroyl sarcosinate, sodium myristoyl sarosinate, sodium palmitoyl sarosinate, sodium lauroyl collagen and palmitoyl keratin.

10. Composition according to Claim 5, characterized in that the said alkyl or alkenyl ether or ester sulphate salts are chosen from sodium lauryl ether sulphate and sodium cocoyl isethionate.

11. Composition according to Claim 5, characterized in that the said polyoxyethylenated alkyl or alkenyl fatty esters or ethers contain at least 20 units of ethylene oxide.

12. Composition according to Claim 11, characterized in that the said polyoxyethylenated alkyl or alkenyl fatty esters or ethers are chosen from PEG-20 stearate, laureth-23, oleth-20 and PEG-25 phytosterol.

13. Composition according to Claim 5, characterized in that the said polyoxyethylenated alkyl or alkenyl carboxylic acids and salts thereof contain at least 10 units of ethylene oxide.

14. Composition according to Claim 13, characterized in that the said polyoxyethylenated alkyl or alkenyl carboxylic acids are chosen from laureth-10 carboxylic acid and oleth-10 carboxylic acid.

15. Composition according to Claim 5, characterized in that the said N-alkyl or N-alkenyl betaines are chosen from laurylamidopropyl betaine and oleylamidopropyl betaine.

16. Composition according to Claim 5, characterized in that the said alkyltrimethylammonium or alkenyltrimethylammonium salts are chosen from cocoyltrimethylammonium chloride and cetyltrimethylammonium bromide.

17. Composition according to any one of the preceding claims, characterized in that the said cubic gel particles additionally contain from 0.0005% to 5% by weight of at least one water-insoluble ionic amphiphilic lipid relative to the total weight of the composition.

18. Composition according to Claim 17, characterized in that the proportion of water-insoluble ionic amphiphilic lipid is from 0.001% to 2% by weight relative to the total weight of the composition.

19. Composition according to either of Claims 17 and 18, characterized in that the said water-insoluble ionic amphiphilic lipid is chosen from:
(i)phospholipids,
(ii) fatty acid phosphonic esters,
(iii) water-insoluble N-acylated derivatives of glutamic acid,
(iv) sodium cetyl sulphate,
(v)sodium cocoyl monoglyceride sulphate, and
(vi) water-insoluble quaternary ammonium derivatives.

20. Composition according to Claim 19, characterized in that the said phospholipid is chosen from soya lecithin, egg lecithin, hydrogenated lecithin, phosphatidic acid sodium salt and dipalmitoylphosphatidylcholine.

21. Composition according to Claim 19, characterized in that the said fatty acid phosphonic ester is chosen from monocetyl phosphate and dimyristyl phosphate and the sodium and potassium salts thereof.

22. Composition according to Claim 19, characterized in that the said water-insoluble N-acylated glutamic acid derivative is chosen from monosodium steroyl glutamate and the monosodium cocoyl- (C₁₄-C₂₀)alkoyl glutamate mixture, the C₁₄-C₂₀ alkoyl radical being derived from hydrogenated tallow.

23. Composition according to Claim 19, characterized in that the said water-insoluble quaternary ammonium derivative is chosen from behenyltrimethylammonium chloride, dilauryldimethylammonium chloride, distearyldimethylammonium chloride, 4,5-dihydro-1-methyl-2-(C₁₄-C₂₀)alkoyl-1-(2- (C₁₄-C₂₀)alkoylaminoethyl) imidazolium methyl sulphate in which the C₁₄-C₂₀ alkoyl radicals are derived from hydrogenated tallow, dialkylhydroxyethylmethylammonium methyl sulphate in which the alkyl radicals are derived from tallow, which may or may not be hydrogenated, and quaternium-82.

24. Composition according to any one of the preceding claims, characterized in that the said particles have an average size approximately from 0.05 µm to 1 µm.

25. composition according to any one of the preceding claims, characterized in that the said particles have an average size less than or equal to 0.5 µm.

26. Composition according to any one of the preceding claims, characterized in that the said particles contain at least one hydrophilic active principle.

27. Composition according to any one of the preceding claims, characterized in that the said particles contain at least one lipophilic active principle.

28. Composition according to any one of the preceding claims, characterized in that the said particles contain both at least one hydrophilic active principle and at least one lipophilic active principle.

29. Composition according to any one of the preceding claims, characterized in that it additionally contains in the aqueous phase at least one cosmetically or dermatologically acceptable compound chosen from a hydrophilic active principle, a preserving agent, a fragrance, a pigment, a dyestuff, a filler and a gelling agent.

30. Composition according to any one of the preceding claims, characterized in that it additionally comprises liposomes.

31. Use of a composition as defined according to any one of the preceding claims for hydrating the skin by topical application.

32. Cosmetic method for hydrating the skin, characterized in that it consists in applying a composition as defined according to any one of Claims 1 to 30, optionally containing a cosmetic active principle, to the skin.
